# EUROPÄISCHE PATENTSCHRIFT

(11) **EP 3 185 799 B1**
(45) Veröffentlichungstag und Bekanntmachung des Hinweises auf die Patenterteilung: **26.05.2021**
(21) Anmeldenummer: 15748047.6
(22) Anmeldetag: 11.08.2015
(51) Int. Cl.: A61B 18/14, A61B 90/90, A61B 90/92, A61B 90/94, A61B 90/96

(54) **ELEKTROCHIRURGISCHES SYSTEM UND VERFAHREN ZUM BETREIBEN DESSELBEN**
ELECTRIC SURGICAL SYSTEM AND METHOD FOR OPERATING SAME
SYSTÈME ÉLECTROCHIRURGICAL ET PROCÉDÉ POUR FAIRE FONCTIONNER CELUI-CI

(30) Priorität: 27.08.2014 DE 102014217095
(43) Veröffentlichungstag der Anmeldung: 05.07.2017
(73) Patentinhaber: Olympus Winter & Ibe GmbH, 22045 Hamburg (DE)
(72) Erfinder: HIRSCHFELD, Simon, 21360 Vögelsen (DE)
(74) Vertreter: Seemann & Partner Patentanwälte mbB
(86) Internationale Anmeldenummer: PCT/EP2015/068419
(87) Internationale Veröffentlichungsnummer: WO 2016/030178

(56) Entgegenhaltungen:
- EP-A1- 2 008 598
- EP-A1- 2 329 783
- EP-A2- 2 110 698
- EP-A2- 2 641 552
- DE-A1-102009 017 616
- US-A- 5 649 021
- US-A1- 2008 262 654
- US-A1- 2013 046 299

## Beschreibung

Die Erfindung betrifft ein elektrochirurgisches System, umfassend ein chirurgisches Instrument mit einem Bilderfassungssystem und einem Schaft, wobei an einem distalen Ende des Schafts eine Elektrodenaufnahme vorhanden ist, und wobei das Bilderfassungssystem zum Erfassen eines Operationsfeldes eingerichtet ist, welches sich distal vor dem distalen Ende des Schafts erstreckt. Ferner betrifft die Erfindung ein Verfahren zum Betreiben eines solchen elektrochirurgischen Systems.

In der Hochfrequenzchirurgie (auch als HF-Chirurgie bezeichnet) wird ein hochfrequenter Wechselstrom durch chirurgisch zu behandelndes Gewebe geleitet, um dieses gezielt zu schädigen oder zu schneiden. Der wesentliche Vorteil dieser Operationstechnik gegenüber der Verwendung eines herkömmlichen Skalpells ist, dass gleichzeitig mit dem Schnitt eine Blutungsstillung durch Verschluss der betroffenen Gefäße erfolgt. Bei der Hochfrequenzchirurgie wird eine anstatt eines Skalpells verwendete Elektrode auf ein chirurgisches Instrument, beispielsweise auf ein Resektoskop, aufgesteckt. Zum Betreiben der Elektrode ist es wichtig, dass diese mit den vorgesehenen Betriebsparametern betrieben wird.

Die Einstellung der korrekten Betriebsparameter wird für den Operateur dadurch erleichtert, dass das chirurgische Instrument in der Lage ist, die aufgesteckte Elektrode selbstständig zu erkennen. Zu diesem Zweck befindet sich beispielsweise ein integrierter Schaltkreis (IC) im Stecker der Elektrode. Es ist jedoch technisch aufwendig und kostenintensiv solche ICs in die Elektrode zu integrieren. Da es sich bei den Elektroden aus hygienischen Gründen teilweise um Einwegartikel handelt, tragen die Kosten der Elektroden direkt zu den Betriebskosten des elektrochirurgischen Systems bei.

Aus DE 10 2009 017 616 A1 ist ein System zur RF-Ablation bekannt, bei dem eine Behandlungssonde in ein zu behandelndes Gewebe eingeführt wird und gleichzeitig eine CT erstellt wird. Das zur Behandlung eingesetzte Ablationsgerät umfasst mehrere Ablationssonden. Um festzustellen, welche der Ablationssonden sich in welchem Gewebebereich befindet, können die einzelnen Ablationssonden im CT-Bild anhand ihrer Materialstärke unterschieden werden. Im CT-Bild ist außerdem der Erfolg der RF-Behandlung feststellbar. Durch Rückkopplung der aus dem CT-Bild gewonnenen Messwerte und iterative Verfeinerung der Steuerbefehle für die einzelnen Sonden, ist es möglich, den Ablationsprozess bei individueller Ansteuerung der Ablationssonden zu regeln.

Aus der EP 2 641 552 A2 ist ein elektrochirurgisches Instrument bekannt, welches an einem distalen Ende eines Schafts eine Zange aufweist, mit der eine RF-Behandlung durchführbar ist. Unterschiedliche Typen dieser Behandlungsgeräte sind mit einem Farbcode codiert.

Aus der EP 2 008 598 A1 ist ein chirurgisches Instrument für eine elektrochirurgische Behandlung bekannt, wobei eine Vielzahl unterschiedlicher Spitzen auf einen Hauptteil des chirurgischen Instruments aufgesetzt werden kann. Die Spitzen sind farblich codiert und befinden sich in entsprechend farblich codierten Aufnahmen.

Ausgehend von diesem Stand der Technik ist es eine Aufgabe der Erfindung, ein elektrochirurgisches System und ein Verfahren zum Betreiben eines elektrochirurgisches Systems anzugeben, wobei eine vereinfachte Erkennung einer mit dem elektrochirurgischen System verbundenen Elektrode möglich sein soll.

Die Aufgabe wird gelöst durch ein elektrochirurgisches System, umfassend ein chirurgisches Instrument mit einem Bilderfassungssystem und einem Schaft, wobei an einem distalen Ende des Schafts eine Elektrodenaufnahme vorhanden ist, und wobei das Bilderfassungssystem zum Erfassen eines Operationsfeldes eingerichtet ist, welches sich distal vor dem distalen Ende des Schafts erstreckt, wobei das System ferner umfasst:
- eine mit der Elektrodenaufnahme verbindbare Elektrode zum Durchführen einer chirurgischen Behandlung, wobei die Elektrode ein optisch erfassbares äußeres Identifikationsmerkmal umfasst,
- eine Datenverarbeitungseinheit mit einer Datenbank, die eine Vielzahl von Datensätzen umfasst, in denen jeweils Informationen betreffend zumindest ein Identifikationsmerkmal eines Elektrodentyps und zumindest ein zum Betreiben dieses Elektrodentyps vorgesehener Betriebsparameter einander zugeordnet sind,
   wobei
- das Bilderfassungssystem dazu eingerichtet ist, ein Bild der Elektrode zu erfassen und Bilddaten des erfassten Bildes an die Datenverarbeitungseinheit zu übertragen,
- die Datenverarbeitungseinheit dazu eingerichtet ist, die empfangenen Bilddaten hinsichtlich eines in den Bilddaten vorhandenen Identifikationsmerkmals zu analysieren,
- ein aufgefundenes Identifikationsmerkmal mit den in der Datenbank vorhandenen Datensätzen abzugleichen,
- einen Typ der Elektrode zu identifizieren und den zugehörigen zumindest einen Betriebsparameter an eine Steuerung des chirurgischen Instruments zu übertragen und wobei
- das Identifikationsmerkmal ein auf einer Außenfläche der Elektrode vorhandener maschinenlesbarer Code ist.

Die Erfindung beruht auf der Erkenntnis, dass die Betriebskosten eines elektrochirurgischen Systems gesenkt werden, wenn zur Erkennung eines Typs der verwendeten Elektrode das vorhandene Bilderfassungssystem des chirurgischen Instruments genutzt wird.

Ferner wird ein äußeres Identifikationsmerkmal der Elektrode verwendet, um deren Typ zu erkennen. Vorteilhaft wird auf eine elektronische Kennzeichnung der Elektroden, beispielsweise einen IC im Stecker der Elektrode, verzichtet. Diese Maßnahmen senken die Stückkosten der Elektroden und folglich auch die Betriebskosten des elektrochirurgischen Systems.

Insbesondere ist vorgesehen, dass nach Übertragung der Betriebsparameter an eine Steuerung des chirurgischen Instruments das elektrochirurgische System auch mit diesen Betriebsparametern betrieben wird.

Gemäß einer vorteilhaften Ausführungsform ist ferner vorgesehen, dass das Identifikationsmerkmal außerdem zumindest ein Teil einer äußeren Form der Elektrode ist.

Ein Teil der äußeren Form sind beispielsweise Abmessungen und/oder Größenverhältnisse einzelner Bauteile der Elektrode. Ferner sind als Identifikationsmerkmale vorhandene Kanten, Fasen, Anordnungen von Verbindungs- oder Fügenähten etc. geeignet. Als maschinenlesbarer Code ist beispielsweise ein Barcode oder ein QR-Code oder ebenso ein Farbcode einsetzbar. Als Farbcode werden bevorzugt Farbpunkte eingesetzt, die maschinell aufgebracht werden. Ein Strichcode oder QR-Code ist insbesondere mit einer Laserbeschriftungseinrichtung auf die Elektrode aufgebracht. Vorteilhaft ist die Kennzeichnung der Elektrode mit geringem Aufwand durchführbar, was ihre Herstellungskosten senkt.

Gemäß einem weiteren Ausführungsbeispiel ist vorgesehen, dass die Elektrode umfasst:
- einen aktiven Bereich, der während der Durchführung der chirurgischen Behandlung in Kontakt mit zu behandelndem Gewebe steht und
- einen zum elektrischen Kontaktieren der Elektrode vorgesehenen Anschlussbereich mit zumindest einem elektrischen Anschlusskontakt,
wobei der zumindest eine elektrische Anschlusskontakt und der aktive Bereich über eine von außen sichtbare elektrische Zuleitung miteinander verbunden und
wobei eine Farbe und/oder ein Farbcode der elektrischen Zuleitung, insbesondere einer elektrischen Isolierung der Zuleitung, das Identifikationsmerkmal sind.

Abhängig von den Betriebsparametern der verwendeten Elektrode, so wie beispielsweise die zum Betrieb vorgesehene Stromstärke und/oder Spannung, werden unterschiedlich dimensionierte elektrische Zuleitungen zum Kontaktieren der Anschlusskontakte mit dem aktiven Bereich verwendet. Diese elektrischen Zuleitungen sind typischerweise mit unterschiedlichen Farben oder Farbcodes markiert, so dass sie während der Herstellung der Elektrode identifizierbar sind. Diese Information, welche herstellerseitig bei der Herstellung der Elektrode in Form der verwendeten elektrischen Zuleitungen bereits integriert wird, ist vorteilhaft nutzbar, um zu einem späteren Zeitpunkt den Typ der Elektrode festzustellen. Besonders vorteilhaft ist, dass zur Kennzeichnung der Elektrode keine zusätzlichen Schritte in den Herstellungsprozess integriert werden müssen.

Gemäß einer weiteren vorteilhaften Ausführungsform ist vorgesehen, dass der zumindest eine Betriebsparameter eine zum Betreiben der Elektrode vorgesehene Betriebsspannung und/oder eine Betriebsstromstärke und/oder eine Betriebsfrequenz und/oder eine maximale Betriebsspannung und/oder eine maximale Betriebsstromstärke und/oder eine maximale Betriebsfrequenz umfasst.

Insbesondere ist das chirurgische Instrument ein Endoskop. Weiterhin insbesondere handelt es sich bei dem chirurgischen Instrument um ein Resektoskop und bei der Elektrode um eine Resektionselektrode.

Als Elektrode sind sowohl monopolare als auch bipolare Elektroden vorgesehen.

Die erfindungsgemäße Aufgabe wird ferner gelöst durch ein Verfahren zum Betreiben eines elektrochirurgischen Systems nach einem oder mehreren der genannten Ausführungsformen, wobei das Verfahren die folgenden Schritte umfasst:
- das Bilderfassungssystem erfasst ein Bild der Elektrode und überträgt die Bilddaten des erfassten Bildes an die Datenverarbeitungseinheit,
- die Datenverarbeitungseinheit analysiert die empfangenen Bilddaten hinsichtlich eines in den Bilddaten vorhandenen Identifikationsmerkmals,
- gleicht ein aufgefundenes Identifikationsmerkmal mit den in der Datenbank vorhandenen Datensätzen ab,
- identifiziert einen Typ der Elektrode und überträgt den zugehörigen zumindest einen Betriebsparameter an eine Steuerung des chirurgischen Instruments
wobei die Datenverarbeitungseinheit in den Bilddaten einen auf einer Außenfläche der Elektrode vorhandenen maschinenlesbaren Code als Identifikationsmerkmal analysiert.

Insbesondere wird das elektrochirurgische System auch mit den von der Datenverarbeitungseinheit an die Steuerung übertragenen Parametern betrieben.

Gemäß einer Ausführungsform ist das Verfahren dadurch fortgebildet, dass die Datenverarbeitungseinheit in den Bilddaten eine Farbe und/oder einen Farbcode einer elektrischen Zuleitung als maschinenlesbaren Code analysiert.

Ferner ist insbesondere vorgesehen, dass die Datenverarbeitungseinheit in den Bilddaten zusätzlich einen Teil einer äußeren Form der Elektrode als Identifikationsmerkmal analysiert.

Ferner ist insbesondere vorgesehen, dass als Betriebsparameter eine zum Betreiben der Elektrode vorgesehene Betriebsspannung und/oder eine Betriebsstromstärke und/oder eine Betriebsfrequenz und/oder eine maximale Betriebsspannung und/oder eine maximale Betriebsstromstärke und/oder eine maximale Betriebsfrequenz von der Datenverarbeitungseinheit an die Steuerung des chirurgischen Instruments übertragen werden.

Auf das Verfahren zum Betreiben des elektrochirurgischen Systems treffen gleiche oder ähnliche Vorteile zu, wie sie bereits im Hinblick auf das elektrochirurgische System selbst erwähnt wurden, so dass sie an dieser Stelle nicht wiederholt werden sollen.

Eine beispielhafte Elektrode für ein elektrochirurgisches System umfasst einen auf einer Außenfläche der Elektrode vorhandenen maschinenlesbaren Code als optisch erfassbares äußeres Identifikationsmerkmal.

Als maschinenlesbarer Code sind beispielsweise ein Barcode und/oder ein QR-Code geeignet, welcher insbesondere mithilfe eines Lasergravurverfahrens auf die Oberfläche der Elektrode aufgebracht wird. Ferner ist ein maschinell aufgebrachter Farbcode, insbesondere bestehend aus Farbpunkten, als Identifikationsmerkmal geeignet.

Als zusätzliches äußeres Identifikationsmerkmal der Elektrode ist ferner insbesondere eine besondere äußere Form der Elektrode, beispielsweise eine äußere Abmessung, Größenverhältnisse, eine Länge eines bestimmten Bauteils, eine Kante und/oder Fase, geeignet. Eines oder mehrere dieser Merkmale werden vorteilhaft als Identifikationsmerkmal der Elektrode verwendet.

Weitere Merkmale der Erfindung werden aus der Beschreibung erfindungsgemäßer Ausführungsformen zusammen mit den Ansprüchen und den beigefügten Zeichnungen ersichtlich. Erfindungsgemäße Ausführungsformen können einzelne Merkmale oder eine Kombination mehrerer Merkmale erfüllen.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:
- Fig. 1: ein elektrochirurgisches System in schematischer Darstellung,
- Fig. 2: eine schematische Perspektivansicht eines Bereichs eines distalen Schaftendes eines Resektoskops mit angeschlossener Elektrode und
- Fig. 3: eine weitere schematische Perspektivansicht eines distalen Schaftendes eines weiteren Resektoskops mit angeschlossener weiterer Elektrode.

In den Zeichnungen sind jeweils gleiche oder gleichartige Elemente und/oder Teile mit denselben Bezugsziffern versehen, so dass von einer erneuten Vorstellung jeweils abgesehen wird.

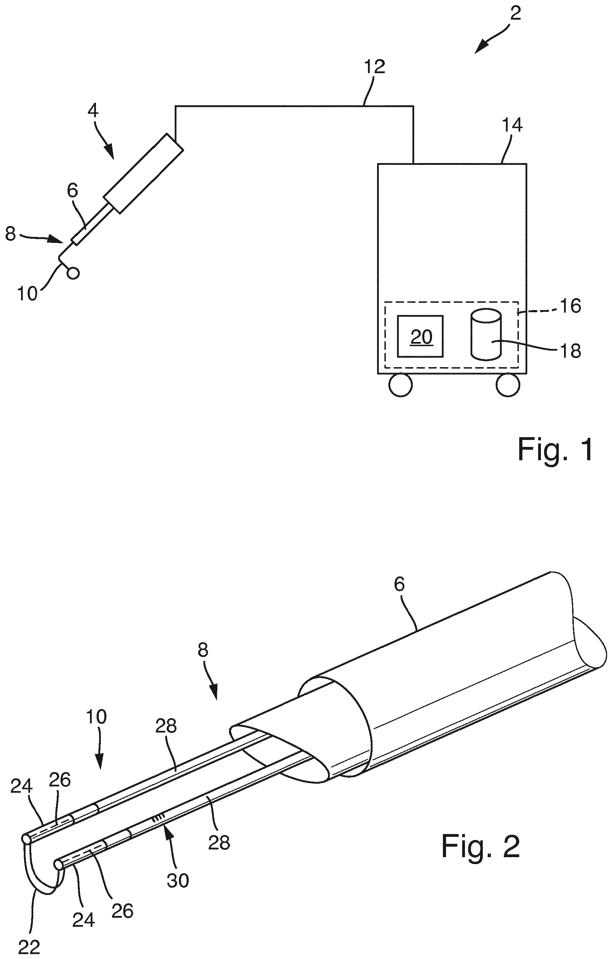

Fig. 1 zeigt in schematischer vereinfachter Darstellung ein elektrochirurgisches System 2, umfassend ein chirurgisches Instrument 4 mit einem nicht dargestellten Bilderfassungssystem und einem Schaft 6. An einem distalen Ende 8 des Schafts 6 ist eine Elektrodenaufnahme vorhanden. Diese dient der Aufnahme einer Elektrode 10 zum Durchführen einer chirurgischen Behandlung.

Beispielhaft ist in Fig. 1 ein Resektoskop als chirurgisches Instrument 4 dargestellt. Entsprechend ist die Elektrode 10 eine Resektionselektrode, d.h. eine Schlinge.

Das chirurgische Instrument 4 ist über eine Versorgungsleitung 12 mit einer Steuereinheit 14 verbunden. Die Versorgungsleitung 12 dient der elektrischen Versorgung des chirurgischen Instruments 4 und auch der Datenübertragung zwischen der Steuereinheit 14 und dem chirurgischen Instrument 4.

Die Steuereinheit 14 umfasst neben anderen allgemein bekannten Einheiten, die nicht dargestellt sind, eine Datenverarbeitungseinheit 16, beispielsweise einen PC mit einer Datenbank 18. In der Datenbank 18, die beispielsweise auf einem nichtflüchtigen Datenspeicher abgelegt ist, befinden sich eine Vielzahl von Datensätzen. Jeder dieser Datensätze enthält Informationen betreffend zumindest ein Identifikationsmerkmal eines Elektrodentyps und zumindest ein zum Betreiben dieses Elektrodentyps vorgesehen Betriebsparameter. Diese beiden Informationen sind einander zugeordnet.

Das Bilderfassungssystem des chirurgischen Instruments 4 ist dazu eingerichtet, ein Bild der Elektrode 10 zu erfassen und die zugehörigen Bilddaten des erfassten Bildes an die Datenverarbeitungseinheit 16 zu übertragen. Die Datenübertragung erfolgt beispielsweise über die Versorgungsleitung 12. Sie kann ebenso auf anderem Wege, beispielsweise drahtlos, erfolgen. Die Datenverarbeitungseinheit 16 ist dazu eingerichtet, die empfangenen Bilddaten hinsichtlich eines in den Bilddaten vorhandenen Identifikationsmerkmals zu analysieren. Findet die Datenverarbeitungseinheit 16 in den Bilddaten ein Identifikationsmerkmal auf, so wird dieses mit den in der Datenbank 18 vorhandenen Datensätzen verglichen. Genauer gesagt findet ein Vergleich mit den in den Datensätzen vorhandenen Informationen betreffend verschiedene Identifikationsmerkmale verschiedener Elektrodentypen statt. Die Datenverarbeitungseinheit 16 identifiziert so den Typ der Elektrode 10, dessen Bild erfasst wurde. Sie ruft die zugehörigen Betriebsparameter für diesen Elektrodentyp aus dem zugehörigen Datensatz der Datenbank 18 ab. Diese Betriebsparameter werden an eine Steuerung 20 des chirurgischen Instruments 4 übertragen. Anschließend erfolgt der Betrieb des chirurgischen Instruments 4 mit diesen Betriebsparametern. Beispielsweise werden eine passende Stromstärke und eine passende Spannung eingestellt.

Lediglich beispielhaft ist in Fig. 1 die Steuerung 20 des chirurgischen Instruments 4 als Teil der Datenverarbeitungseinheit 16 dargestellt.

In dem beschriebenen elektrochirurgischen System 2 wird die Steuerung 20 also automatisch auf die für die Elektrode 10 korrekten Betriebsparameter eingestellt. Hierbei wird der Erfahrungssatz ausgenutzt, dass bevor die Elektrode 10 mit der am distalen Ende 8 des Schafts 6 vorhandenen Elektrodenaufnahme verbunden wird, sich die Elektrode 10 zumindest kurzzeitig im Blickfeld des Bilderfassungssystems des chirurgischen Instruments 4 befindet. So ist es ohne zusätzliche Arbeitsschritte für den Benutzer möglich, ein Bild der Elektrode 10 zu erfassen und auszuwerten.

Sollte das auf diese Weise erfasste Bild nicht ausreichend sein, beispielweise das Identifikationsmerkmal der Elektrode 10 nicht sichtbar sein, so ist die Steuereinheit 14 ggf. dazu eingerichtet, an den Benutzer des chirurgischen Instruments 4 eine Information auszugeben, die Elektrode 10 in das Blickfeld des Bilderfassungssystems zu verbringen. Beispielsweise wird eine visuelle oder akustische Aufforderung ausgegeben.

Fig. 2 zeigt in vereinfachter, schematisch perspektivischer Ansicht einen distalen Endbereich des Schafts 6 des chirurgischen Instruments 4. Die Elektrode 10 umfasst einen aktiven Bereich 22, welcher in Form einer Schlinge ausgebildet ist. Beispielhaft ist eine bipolare Elektrode 10 gezeigt. Das elektrochirurgische System 2 ist jedoch ebenso für monopolare Elektroden eingerichtet. Der aktive Bereich 22 steht bei der Durchführung einer chirurgischen Behandlung in Kontakt mit dem zu behandelnden Gewebe.

An den aktiven Bereich 22 schließt sich ein Übergangsbereich an, welcher aus einem Paar transparenter Röhrchen 24 und darin geführten elektrischen Zuleitungen 26 aufgebaut ist. In proximaler Richtung schließt sich an diesen Übergangsbereich ein Anschlussbereich 28 der Elektrode 10 an. An einem in Fig. 2 nicht sichtbaren proximalen Ende des Anschlussbereichs 28 befindet sich ein elektrischer Anschlusskontakt bzw. Stecker. Dieser wird zum Herstellen eines elektrischen Kontakts in die Elektrodenaufnahme des Schafts 6 des chirurgischen Instruments 4 eingesteckt.

Die elektrischen Zuleitungen 26 sind von außen durch die transparenten Röhrchen 24 sichtbar. Je nach Typ der Elektrode 10 werden bei ihrer Herstellung unterschiedlich farbige elektrische Zuleitungen 26 verwendet. Ebenso ist vorgesehen, die elektrischen Zuleitungen 26 mit zu diesem Zweck ausgewählten unterschiedlichen Farbcodes zu kodieren.

So ist es möglich, die Elektrode 10 anhand der Farbe und/oder des Farbcodes der elektrischen Zuleitungen 26 ihrem Typen nach zu identifizieren. Dieser Farbe oder dem Farbcode kommt also die Rolle eines Identifikationsmerkmals zu.

Ferner ist vorgesehen, dass eine äußere Form der Elektrode 10 oder ein auf einer Außenfläche der Elektrode 10 vorhandener maschinenlesbarer Code 30 als Identifikationsmerkmal verwendet werden. Bei dem maschinenlesbaren Code 30 handelt es sich beispielsweise um einen Barcode und/oder um einen QR-Code. Dieser ist insbesondere mit einem Lasergravurverfahren auf die Elektrode 10 aufgebracht.

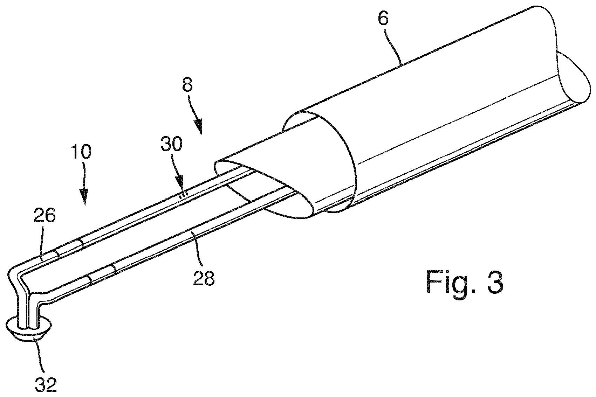

Fig. 3 zeigt in einer weiteren schematisch vereinfachten und perspektivischen Darstellung einen Teil des Schafts 6 des chirurgischen Instruments 4 im Bereich seines distalen Endes 8. Die von der Elektrodenaufnahme am distalen Ende 8 des Schafts 6 aufgenommene Elektrode 10 ist keine Elektroschlinge, so wie sie in Fig. 2 gezeigt ist, sondern eine Elektrode 10 mit einer Knopfelektrode 32. Diese ist über zwei elektrische Zuleitungen 26 mit dem Anschlussbereich 28 der Elektrode 10 verbunden. Neben einem maschinenlesbaren Code 30 und der Form der Elektrode 10 ist eine Farbe und/oder ein Farbcode der Isolierungen der Zuleitungen 26 als Identifikationsmerkmal für die Elektrode 10 vorgesehen.

Nachdem der Typ der Elektrode 10 identifiziert wurde, werden der Steuerung 20 als Betriebsparameter beispielsweise eine zum Betreiben der Elektrode 10 vorgesehene Betriebsspannung und/oder eine Betriebsstromstärke und/oder eine Betriebsfrequenz zugeführt. Ebenso ist vorgesehen, die entsprechenden Maximalwerte der Betriebsspannung und/oder der Betriebsstromstärke und/oder eine maximale Betriebsfrequenz als Betriebsparameter zu übergeben.

Alle genannten Merkmale, auch die den Zeichnungen allein zu entnehmenden sowie auch einzelne Merkmale, die in Kombination mit anderen Merkmalen offenbart sind, werden allein und in Kombination als erfindungswesentlich angesehen. Erfindungsgemäße Ausführungsformen können durch einzelne Merkmale oder eine Kombination mehrerer Merkmale erfüllt sein. Im Rahmen der Erfindung sind Merkmale, die mit "insbesondere" oder "vorzugsweise" gekennzeichnet sind, als fakultative Merkmale zu verstehen.

### Bezugszeichenliste

- 2: elektrochirurgisches System
- 4: chirurgisches Instrument
- 6: Schaft
- 8: distales Ende
- 10: Elektrode
- 12: Versorgungsleitung
- 14: Steuereinheit
- 16: Datenverarbeitungseinheit
- 18: Datenbank
- 20: Steuerung
- 22: aktiver Bereich
- 24: transparentes Röhrchen
- 26: elektrische Zuleitung
- 28: Anschlussbereich
- 30: maschinenlesbarer Code
- 32: Knopflelektrode

## Patentansprüche

1. Elektrochirurgisches System (2), umfassend ein chirurgisches Instrument (4) mit einem Bilderfassungssystem und einem Schaft (6), wobei an einem distalen Ende (8) des Schafts (6) eine Elektrodenaufnahme vorhanden ist, und wobei das Bilderfassungssystem zum Erfassen eines Operationsfeldes eingerichtet ist, welches sich distal vor dem distalen Ende (8) des Schafts (6) erstreckt, wobei das System ferner umfasst:
- eine mit der Elektrodenaufnahme verbindbare Elektrode (10) zum Durchführen einer chirurgischen Behandlung, wobei die Elektrode (10) ein optisch erfassbares äußeres Identifikationsmerkmal umfasst,
- eine Datenverarbeitungseinheit (16) mit einer Datenbank (18), die eine Vielzahl von Datensätzen umfasst, in denen jeweils Informationen betreffend zumindest ein Identifikationsmerkmal eines Elektrodentyps und zumindest ein zum Betreiben dieses Elektrodentyps vorgesehener Betriebsparameter einander zugeordnet sind,
wobei
- das Bilderfassungssystem dazu eingerichtet ist, ein Bild der Elektrode (10) zu erfassen und Bilddaten des erfassten Bildes an die Datenverarbeitungseinheit (16) zu übertragen,
- die Datenverarbeitungseinheit (16) dazu eingerichtet ist, die empfangenen Bilddaten hinsichtlich eines in den Bilddaten vorhandenen Identifikationsmerkmals zu analysieren,
- ein aufgefundenes Identifikationsmerkmal mit den in der Datenbank (18) vorhandenen Datensätzen abzugleichen,
- einen Typ der Elektrode (10) zu identifizieren und den zugehörigen zumindest einen Betriebsparameter an eine Steuerung (20) des chirurgischen Instruments (4) zu übertragen
und wobei
das Identifikationsmerkmal ein auf einer Außenfläche der Elektrode (10) vorhandener maschinenlesbarer Code (30) ist.

2. Elektrochirurgisches System (2) nach Anspruch 1, **dadurch gekennzeichnet, dass** das Identifikationsmerkmal außerdem zumindest ein Teil einer äußeren Form der Elektrode (10) ist.

3. Elektrochirurgisches System (2) nach Anspruch 1 oder 2, **dadurch gekennzeichnet, dass** die Elektrode (10) umfasst:
- einen aktiven Bereich (22), der während der Durchführung der chirurgischen Behandlung in Kontakt mit zu behandelndem Gewebe steht und
- einen zum elektrischen Kontaktieren der Elektrode (10) vorgesehenen Anschlussbereich (28) mit zumindest einem elektrischen Anschlusskontakt,
wobei der zumindest eine elektrische Anschlusskontakt und der aktive Bereich (22) über eine von außen sichtbare elektrische Zuleitung (26) miteinander verbunden und
wobei eine Farbe und/oder ein Farbcode der elektrischen Zuleitung (26), insbesondere einer elektrischen Isolierung der Zuleitung (26), das Identifikationsmerkmal sind.

4. Elektrochirurgisches System (2) nach einem der Ansprüche 1 bis 3, **dadurch gekennzeichnet, dass** der zumindest eine Betriebsparameter eine zum Betreiben der Elektrode (10) vorgesehene Betriebsspannung und/oder eine Betriebsstromstärke und/oder eine Betriebsfrequenz und/oder eine maximale Betriebsspannung und/oder eine maximale Betriebsstromstärke und/oder eine maximale Betriebsfrequenz umfasst.

5. Elektrochirurgisches System (2) nach einem der Ansprüche 1 bis 4, **dadurch gekennzeichnet, dass** das chirurgische Instrument (4) ein Endoskop ist, wobei das chirurgische Instrument (4) insbesondere ein Resektoskop und die Elektrode (10) eine Resektionselektrode sind.

6. Verfahren zum Betreiben eines elektrochirurgischen Systems (2), nach einem der Ansprüche 1 bis 5, wobei das Verfahren die folgenden Schritte umfasst:
- das Bilderfassungssystem erfasst ein Bild der Elektrode (10) und überträgt die Bilddaten des erfassten Bildes an die Datenverarbeitungseinheit (16),
- die Datenverarbeitungseinheit (16) analysiert die empfangenen Bilddaten hinsichtlich eines in den Bilddaten vorhandenen Identifikationsmerkmals,
- gleicht ein aufgefundenes Identifikationsmerkmal mit den in der Datenbank (18) vorhandenen Datensätzen ab,
- identifiziert einen Typ der Elektrode (10) und überträgt den zugehörigen zumindest einen Betriebsparameter an die Steuerung (20) des chirurgischen Instruments (4),
wobei die Datenverarbeitungseinheit (16) in den Bilddaten einen auf einer Außenfläche der Elektrode (10) vorhandenen maschinenlesbaren Code (30) als Identifikationsmerkmal analysiert.

7. Verfahren nach Anspruch 6, **dadurch gekennzeichnet, dass** die Datenverarbeitungseinheit (16) in den Bilddaten eine Farbe und/oder einen Farbcode einer elektrischen Zuleitung (26) als maschinenlesbaren Code (30) analysiert.

8. Verfahren nach Anspruch 6, **dadurch gekennzeichnet, dass** die Datenverarbeitungseinheit (16) in den Bilddaten zusätzlich einen Teil einer äußeren Form der Elektrode (10) als Identifikationsmerkmal analysiert.

9. Verfahren nach einem der Ansprüche 6 bis 8, **dadurch gekennzeichnet, dass** als Betriebsparameter eine zum Betreiben der Elektrode (10) vorgesehene Betriebsspannung und/oder eine Betriebsstromstärke und/oder eine Betriebsfrequenz und/oder eine maximale Betriebsspannung und/oder eine maximale Betriebsstromstärke und/oder eine maximale Betriebsfrequenz von der Datenverarbeitungseinheit (16) an eine Steuerung (20) des chirurgischen Instruments (4) übertragen werden.

## Claims

1. An electrosurgical system (2), comprising a surgical instrument (4) with an imaging system and a shaft (6), wherein an electrode seat is on a distal end (8) of the shaft (6), and wherein the imaging system is configured to capture a surgical field that extends distally in front of the distal end (8) of the shaft (6), wherein the system further comprises:
- an electrode (10) that is connectible to the electrode seat for performing a surgical procedure, wherein the electrode (10) comprises an optically detectable external identifying feature,
- a data processing unit (16) with a database (18) that comprises a plurality of data sets in which information relating to at least one identifying feature of an electrode type and at least one operating parameter provided for operating this electrode type are assigned to each other,
wherein
- the imaging system is configured to capture an image of the electrode (10) and to transfer image data of the captured image to the data processing unit (16),
- the data processing unit (16) is configured to analyze the received image data with regard to an identifying feature in the image data,
- to compare a found identifying feature with the data sets in the database (18),
- to identify a type of electrode (10) and to transmit the associated at least one operating parameter to a controller (20) of the surgical instrument (4),
and wherein
- the identifying feature is a machine-readable code (30) on an external surface of the electrode (10).

2. The electrosurgical system (2) according to claim 1, **characterized in that** the identifying feature is also at least a part of an external shape of the electrode (10).

3. The electrosurgical system (2) according to claim 1 or 2, **characterized in that** the electrode (10) comprises:
- an active region (22) that is in contact with tissue to be treated while performing the surgical procedure, and
- a connecting region (28) provided for electrically contacting the electrode (10) having at least one electrical connecting contact,
wherein the at least one electrical connecting contact and the active region (22) are connected to each other by an electrical lead (26) visible from the outside, and
wherein a color and/or a color code of the electrical lead (26), in particular an electrical insulation of the lead (26), are the identifying feature.

4. The electrosurgical system (2) according to one of claims 1 to 3, **characterized in that** the at least one operating parameter comprises an operating voltage, and/or an operating current strength, and/or an operating frequency, and/or a maximum operating voltage, and/or a maximum operating current strength, and/or a maximum operating frequency provided for operating the electrode (10).

5. The electrosurgical system (2) according to one of claims 1 to 4, **characterized in that** the surgical instrument (4) is an endoscope, wherein the surgical instrument (4) is in particular a resectoscope and the electrode (10) is a resection electrode.

6. A method for operating an electrosurgical system (2) according to one of claims 1 to 5, wherein the method comprises the following steps:
- the imaging system captures an image of the electrode (10) and transmits the image data of the captured image to the data processing unit (16),
- the data processing unit (16) analyzes the received image data with regard to an identifying feature in the image data,
- compares a found identifying feature with the data sets in the database (18),
- identifies a type of electrode (10) and transmits the associated at least one operating parameter to the controller (20) of the surgical instrument (4),
wherein the data processing unit (16) analyzes a machine-readable code (30) on an external surface of the electrode (10) as the identifying feature in the image data.

7. The method according to claim 6, **characterized in that** the data processing unit (16) analyzes in the image data a color and/or a color code of an electrical lead (26) as the machine-readable code (30).

8. The method according to claim 6, **characterized in that** the data processing unit (16) also analyzes in the image data a part of an external shape of the electrode (10) as the identifying feature.

9. The method according to one of claims 6 to 8, **characterized in that** an operating voltage, and/or an operating current strength, and/or an operating frequency, and/or a maximum operating voltage, and/or a maximum operating current strength, and/or a maximum operating frequency provided for operating the electrode (10) are transmitted from the data processing unit (16) to a controller (20) of the surgical instrument (4) as an operating parameter.

## Revendications

1. Système électro-chirurgical (2) comprenant un instrument chirurgical (4) ayant un système d'acquisition d'images et un arbre (6), un réceptacle d'électrode étant prévu à une extrémité distale (8) de l'arbre (6), et le système d'acquisition d'images étant adapté pour capturer un champ chirurgical s'étendant distalement en avant de l'extrémité distale (8) de l'arbre (6), le système comprenant en outre :
- une électrode (10) apte à être connectée au réceptacle d'électrode pour réaliser une procédure chirurgicale, l'électrode (10) comprenant une caractéristique d'identification externe détectable optiquement,
- une unité (16) de traitement de données ayant une base de données (18) comprenant une pluralité d'ensembles de données dans chacun desquels des informations concernant au moins une caractéristique d'identification d'un type d'électrode et au moins un paramètre de fonctionnement prévu pour faire fonctionner ledit type d'électrode sont associées les unes aux autres,
dans lequel
- le système d'acquisition d'images est agencé pour acquérir une image de l'électrode (10) et pour transmettre des données d'image de l'image acquise à l'unité (16) de traitement de données,
- l'unité (16) de traitement de données est agencée pour analyser les données d'image reçues par rapport à une caractéristique d'identification présente dans les données d'image,
- comparer une caractéristique d'identification détectée avec les enregistrements de données présents dans la base de données (18),
- identifier un type de l'électrode (10) et transmettre ledit au moins un paramètre de fonctionnement associé à un contrôleur (20) de l'instrument chirurgical (4),
et dans lequel
la caractéristique d'identification est un code lisible par machine (30) prévu sur une surface extérieure de l'électrode (10).

2. Système électro-chirurgical (2) selon la revendication 1, **caractérisé en ce que** ladite caractéristique d'identification est en outre au moins une partie d'une forme externe de l'électrode (10).

3. Système électro-chirurgical (2) selon la revendication 1 ou la revendication 2, **caractérisé en ce que** l'électrode (10) comprend :
- une zone active (22) en contact avec le tissu à traiter pendant la réalisation de la procédure chirurgicale ; et
- une zone de connexion (28) prévue pour être en contact électrique avec l'électrode (10) et ayant au moins un contact de connexion électrique,
ledit au moins un contact de connexion électrique et la zone active (22) étant reliés l'un à l'autre par l'intermédiaire d'un conducteur électrique (26) qui est visible de l'extérieur, et
une couleur et/ou un code couleur du conducteur électrique (26), en particulier d'une isolation électrique du conducteur (26), étant la caractéristique d'identification.

4. Système électro-chirurgical (2) selon l'une quelconque des revendications 1 à 3, **caractérisé en ce que** ledit au moins un paramètre de fonctionnement comprend une tension de fonctionnement prévue pour le fonctionnement de l'électrode (10) et/ou une intensité de courant de fonctionnement et/ou une fréquence de fonctionnement et/ou une tension de fonctionnement maximale et/ou une intensité de courant de fonctionnement maximale et/ou une fréquence de fonctionnement maximale.

5. Système électro-chirurgical (2) selon l'une quelconque des revendications 1 à 4, **caractérisé en ce que** l'instrument chirurgical (4) est un endoscope, l'instrument chirurgical (4) étant en particulier un résectoscope et l'électrode (10) étant une électrode de résection.

6. Procédé de fonctionnement d'un système électro-chirurgical (2), selon l'une quelconque des revendications 1 à 5, le procédé comprenant les étapes suivantes :
- le système d'acquisition d'image acquiert une image par l'électrode (10) et transmet les données d'image de l'image acquise à l'unité (16) de traitement de données,
- l'unité (16) de traitement de données analyse les données d'image reçues par rapport à une caractéristique d'identification présente dans les données d'image,
- compare une caractéristique d'identification trouvée avec les enregistrements de données présents dans la base de données (18),
- identifie un type de l'électrode (10), et transmet ledit au moins un paramètre de fonctionnement associé au contrôleur (20) de l'instrument chirurgical (4),
l'unité (16) de traitement de données analysant dans les données d'image un code lisible par machine (30) présent sur une surface extérieure de l'électrode (10) en tant que caractéristique d'identification.

7. Procédé selon la revendication 6, **caractérisé en ce que** l'unité (16) de traitement de données analyse dans les données d'image une couleur et/ou un code couleur d'un conducteur électrique (26) en tant que code lisible par machine (30).

8. Procédé selon la revendication 6, **caractérisé en ce que** l'unité (16) de traitement de données analyse en plus dans les données d'image une partie d'une forme extérieure de l'électrode (10) en tant que caractéristique d'identification.

9. Procédé selon l'une des revendications 6 à 8, **caractérisé en ce que**, en tant que paramètres de fonctionnement, une tension de fonctionnement et/ou une intensité de courant de fonctionnement et/ou une fréquence de fonctionnement et/ou une tension de fonctionnement maximale et/ou une intensité de courant de fonctionnement maximale et/ou une fréquence de fonctionnement maximale prévues pour le fonctionnement de l'électrode (10) sont transmises de l'unité (16) de traitement de données à un contrôleur (20) de l'instrument chirurgical (4).
